# EUROPEAN PATENT SPECIFICATION

(11) **EP 1 660 115 B1**
(45) Date of publication and mention of the grant of the patent: **22.06.2011**
(21) Application number: 04763431.6
(22) Date of filing: 23.07.2004
(51) Int. Cl.: A61K 38/18, A61P 7/00

(54) **PHARMACEUTICAL COMBINATION OF G-CSF AND PLGF USEFUL FOR BLOOD STEM CELL**
PHARMAZEUTISCHE KOMBINATION AUS G-CSF UND PLGF FÜR BLUTSTAMMZELLEN
COMBINAISON PHARMACEUTIQUE DE G-CSF ET PLGF CONVENANT A LA MOBILISATION DES CELLULES SOUCHES DU SANG

(30) Priority: 29.07.2003 EP 03017174
(43) Date of publication of application: 31.05.2006
(73) Proprietor: Dompe' S.P.A., 67100 L'Aquila (IT)
(72) Inventor: GIANNI, Alessandro, Massimo, I-20144 Milano (IT); CARLO-STELLA, Carmelo, I-20144 Milano (IT); COLOTTA, Francesco, I-67100 L'Aquila (IT)
(74) Representative: Perani, Aurelio
(86) International application number: PCT/EP2004/008245
(87) International publication number: WO 2005/014023

(56) References cited:
- KADAR J G ET AL: "Technical and safety aspects of blood and marrow transplantation using G-CSF mobilized family donors" TRANSFUSION SCIENCE, PERGAMON PRESS, OXFORD, GB, vol. 17, no. 4, December 1996 (1996-12), pages 611-618, XP004568854 ISSN: 0955-3886
- HATTORI KOICHI ET AL: "Placental growth factor reconstitutes hematopoiesis by recruiting VEGFR1(+) stem cells from bone-marrow microenvironment." NATURE MEDICINE. AUG 2002, vol. 8, no. 8, August 2002 (2002-08), pages 841-849, XP002307301 ISSN: 1078-8956 cited in the application
- CARMELIET P ET AL: "SYNERGISM BETWEEN VASCULAR ENDOTHELIAL GROWTH FACTOR AND PLACENTAL GROWTH FACTOR CONTRIBUTES TO ANGIOGENESIS AND PLASMA EXTRAVASATION IN PATHOLOGICAL CONDITIONS" NATURE MEDICINE, NATURE PUBLISHING, CO, US, vol. 7, no. 5, May 2001 (2001-05), pages 575-583, XP001017902 ISSN: 1078-8956
- DE REVEL THIERRY ET AL: "Effects of granulocyte colony-stimulating factor and stem cell factor, alone and in combination, on the mobilization of peripheral blood cells that engraft lethally irradiated dogs" BLOOD, vol. 83, no. 12, 1994, pages 3795-3799, XP002307302 ISSN: 0006-4971
- CARLO-STELLA CARMELO ET AL: "Defibrotide in combination with granulocyte colony-stimulating factor significantly enhances the mobilization of primitive and committed peripheral blood progenitor cells in mice" CANCER RESEARCH, vol. 62, no. 21, 1 November 2002 (2002-11-01), pages 6152-6157, XP002307303 ISSN: 0008-5472

## Description

### FIELD OF THE INVENTION

This invention regards a combination of biologically active molecules for use in the mobilization of blood stem cells in a patient or subject in need thereof. More specifically, the invention provides a combination of G-CSF and P1GF particularly effective in stimulating the mobilization of peripheral blood progenitor cells (PBPCs) thereby increasing feasibility and efficacy of organ or cell transplantation and of chemo-radiotherapy protocols in tumor patients.

### BACKGROUND OF THE INVENTION

Autologous PBPCs have significantly increased indications, feasibility and efficacy of high-dose chemo-radiotherapy and autologous stem cell transplantation (SCT)^{1,2} in patients with non-Hodgkin lymphoma (NHL),³ relapsed Hodgkin lymphoma (HL),⁴ as well as multiple myeloma (MM).⁵

Allogeneic PBPCs represent the preferred stem cell source for HLA-matched SCT and the unique source for HLA-mismatched allografts^{6,7,8,9,10,11} which is a potentially curative therapy for patients with high-risk leukemias lacking an HLA-matched related or unrelated donor, i.e., approximately 40% of the global population of patients who may benefit of allogeneic transplantation.

Protocols used to mobilize autologous PBPCs in cancer patients include the use of myeloid growth factors alone or during recovery from cytotoxic chemotherapy, with the latter approach allowing optimal PBPC mobilization^{12,13,14}. Mobilization of allogeneic PBPCs from healthy donors is usually achieved by short courses of recombinant human granulocyte colony-stimulating factor (rhG-CSF) in doses ranging from 10 to 20 µg/kg/day^{15,16,17,18}.

Cancer patients autografted with ≥5 x 10⁶ CD34+ cells/kg experience prompt and durable hematopoietic engraftment, whereas those receiving ≤2 x 10⁶ CD34+ cells/kg are at risk for delayed engraftment, engraftment failure or secondary myelodysplasia¹⁹. Therefore, in the setting of autologous SCT, the availability of adequate amounts of CD34+ cells represents an essential prerequisite. Either due to prior extensive chemo-radiotherapy or disease-related factors, a substantial proportion of chemotherapy naïve (10 to 20%) or relapsed/refractory (30 to 40%) cancer patients fail to mobilize optimal amounts of CD34+ cells^{20,21,22}.

The collection of adequate numbers of allogeneic CD34+ cells does not represent a critical issue in recipients of HLA-identical transplants; however, 5 to 15% of normal donors experience poor stem cell mobilization and require increased doses of rhG-CSF and multiple apheretic procedures^{23,24,25}. Recipients of HLA-mismatched allografting require the reinfusion of "mega" doses of T-lymphocyte-depleted CD34+ cells to prevent graft failure and severe GvHD²⁶. Under the standard mobilization regimen, (i.e., a 7 day course of rhG-CSF) donors for HLA-mismatched SCT undergo an average of 4 leukaphereses to collect the target cell dose of CD34+ cells (12 x 10⁶ CD34⁺ cells/kg body weight), with a substantial proportion of donors (20 to 25%) failing to provide the target CD34+ cell dose.

Despite age, sex, schedule of cytokine treatment as well as previous chemo-radiotherapy may affect stem cell mobilization^{27,28,29}, no specific characteristics have been clearly identified as predictive factors for cytokine mobilization. Therefore, any procedure applicable to cancer patients or normal donors, and capable of increasing the yield of circulating progenitors in the absence of added toxicity, is expected to have a profound impact on the feasibility, toxicity and costs both autologous and allogeneic SCT.

Increased PBPC mobilization might be achieved by using molecules capable of interfering with the mechanism(s) regulating hematopoietic stem cell trafficking, i.e., transmigration through the luminal endothelium to extravascular bone marrow spaces in homing and the reverse in mobilization^{30,31,32,33}. One additional approach to enhance PBPC mobilization relies on the use of combinations of cytokines, such as recombinant human (rh) granulocyte-macrophage colony-stimulating factor (rhGM-CSF) plus rhG-CSF³⁴, interleukin-3 (rhIL-3) plus rhG-CSF or rhGM-CSF³⁵, and PIXY-321³⁶. Finally, enhancement of PBPC mobilization might be achieved by incorporating in the standard mobilization regimen early-acting cytokines, such as stem cell factor (rhSCF)^{37,38} of flt-3³⁹ ligand, capable of expanding marrow progenitors, thus increasing the number of cells susceptible to subsequent mobilization by rhG-CSF.

So far, substitutes or adjuncts to rhG-CSF either failed to substantially improve the mobilization of blood progenitors achieved with rhG-CSF alone, or resulted in a limited improvement outweighed by a substantially increased toxicity.

Placental growth factor (P1GF) is a member of the vascular endothelial growth factor (VEGF) family and functions as an angiogenic amplifier by signaling through VEGF receptor-1 (VEGFR1). Recently, administration of an adenoviral vector expressing human (h) P1GF has been shown to exert complex hematopoietic effects, including enhancement of bone marrow recovery following myelosuppression, and mobilization of hematopoietic progenitors. However, the administration of growth factors following injection of recombinant adenoviral vectors presents several major differences from the direct injection of a purified factor, and might not be predictive of its effects when administered according to the modalities used in the clinical setting.

### DESCRIPTION OF THE INVENTION

Due to the relevant clinical impact of any procedure capable to improve stem cell mobilization, we tested the mobilizing activity of P1GF in animal models allowing to simulate PBPC mobilization as occurring in a clinical situation. Normal BALB/c mice were injected intraperitoneally (IP) for 5 days with either control vehicle (PBS/MSA), rhG-CSF alone (10 µg/d), or a combination of rhG-CSF (10 µg/d) and recombinant murine (rm)P1GF (2.5-5 µg/d). Blood samples were collected 2 hours after the last injection of cytokines and the following parameters were evaluated: white blood cell (WBC) counts, frequency and absolute numbers of colony-forming cells (CFC), absolute numbers of long-term culture-initiating cells (LTC-IC).

The effects of rmP1GF are illustrated in Tables 1-4 below. It is evident that rmP1GF injected alone has no effect on the mobilization of WBC, CFC, and LTC-IC. A 5-day injection of rmP1GF (5 µg/d) combined with rhG-CSF significantly increases mobilization of CFC and LTC-IC, as compared to rhG-CSF alone.

In addition, the mobilizing activity of P1GF/G-CSF combinations was tested in a non-human primate model (Rhesus Monkeys). The results obtained in mice were further confirmed in this animal model. In particular, P1GF/G-CSF combination improved the mobilization of WBCs, CFCs, HPP-CFCs and LTC-ICs, in terms of kinetics, frequency and absolute numbers.

The above-indicated studies have been carried out using procedures and conditions that closely resemble the administration of hematopoietic growth factors to human patients. The results clearly demonstrate the presence of a synergistic effect by hG-CSF and rhP1GF in the mobilization of peripheral blood progenitor cells.

Object of the invention is therefore a combined preparation of G-CSF and P1GF useful for stimulating blood stem cell mobilization in a patient or subject in need thereof. As used herein the terms "patient" and "subject" preferably refer to human individuals, but they may also refer to animals, especially mammals. Examples of states, conditions or diseases that may benefit from the mobilization of blood stem cells include, but are not limited to, organ or cell transplantation and tumor chemo-radiotherapy, in particular autologous^{1,2} or allogeneic SCT in patients with NHL, relapsed HL⁴, MM⁵, or in the recovery phase following myelosuppressive chemotherapy.

The active ingredients of the combined preparation can be simultaneously or separately administered in formulation with pharmaceutically acceptable vehicles and excipients. The parenteral route of administration is preferred. Methods for the preparation of pharmaceutical compositions suitable for parenteral administration are known in the art; details can be found in "Remington: The Science and Practice of Pharmacy", Mack Publishing Co. The amount of active ingredients in the combined preparations according to the invention can be varied depending for instance on the administration route, on the effect sought or condition to be treated, and on the response of the patient. As a general rule, an effective amount of G-CSF and P1GF is able to produce the desired response in terms of blood stem cell mobilization. The patient/subject response can be monitored during the treatment, e.g. by counting the circulating blood stem cells, and if necessary the dosages can be modified accordingly. In a preferred embodiment of the invention, recombinant hG-CSF and rhP1GF are used in form of injectable solutions supplying a daily amount of the active comprised from 1 to 150, preferably from 5 to 20 µg/kg G-CSF and from 10 to 300, preferably from 20 to 150 µg/kg P1GF.

The following examples further illustrate the invention.

### EXAMPLES 1-11 - mobilizing effects of PIGF/G-CSF combination in a mouse model

### MATERIALS AND METHODS

**Animals.** Six- to 8-week-old female BALB/c mice, with body weight of 20 to 25 g, were purchased from Charles River (Milano, Italy, EU). Experimental procedures performed on animals were carried out in accordance with the guidelines of the United Kingdom Coordinating Committee on Cancer Research (UK Coordinating Committee on Cancer Research. UKCCCR guidelines for the welfare of animals in experimental neoplasia. Br. J. Cancer., 58:109-113, 1998.). The mice were injected daily, intraperitoneally (IP), for 5 days with either control vehicle (PBS/MSA), rhG-CSF alone (10 µg/d), or a combination of rhG-CSF (10 µg/d) with recombinant murine (rm)P1GF (2.5-5 µg/d). Each experiment was performed at least on three separate occasions, and three to four mice per group per time point were used.

**Cytokines.** Recombinant human granulocyte colony-stimulating factor (rhG-CSF, Neupogen®) was from Roche (Milan, Italy, EU); rmP1GF was purchased from R&D Systems Inc., Abingdon, United Kingdom); rhP1GF was provided from Geymonat SpA (Anagni, Italy, EU).

**Mobilization protocols.** The standard mobilization protocol included treatment of BA-L-B/c with rhG-CSF-(10 gg/mouse, IP) once daily for 5 days. To evaluate the mobilizing effects of P1GF, rmP1GF (2.5 - 5 µg/mouse, IP) were administered once daily for 5 days either as a single agent or in combination with rhG-CSF.

Controls were injected with PBS/MSA.

**Mobilization parameters.** Mobilization was evaluated by white blood cell (WBC) counts, frequency and absolute numbers of colony-forming cells (CFC), absolute numbers of long-term culture-initiating cells (LTC-IC). Unless otherwise stated, animals were sacrificed two hours after the last treatment.

**Cell harvesting and separation.** PB was harvested from the orbital plexus into heparin containing tubes. After white blood cell (WBC) counting, PB was diluted (1:4, v/v) with PBS and mononuclear cells (MNCs) were separated by centrifugation (280 g, 30 min, room temperature) on a Ficoll discontinuous density gradient. Cells were then washed twice in Iscove's modified Dulbecco's medium (IMDM, Seromed, Berlin, Germany, EU) supplemented with 10% fetal bovine serum (FBS, Stem Cell Technologies, Vancouver, Canada), 2 mM L-glutamine and antibiotics.

**WBC counts.** WBC counts were performed using heparin-anticoagulated blood and an automated counter (ADVIA 120, Bayer, Milano, Italy, EU).

**Colony-forming cell (CFC) assay.** Total colony-forming cells (CFCs), i.e., granulocyte-macrophage colony-forming units (CFU-GM), erythroid burst-forming units (BFU-E), and multilineage CFU (CFU-GEMM) were assessed in standard methylcellulose cultures. Briefly, 1-ml aliquots of blood (5 x 10⁴ to 2 x 10⁵ MNCs) were plated in 35-mm Petri dishes in methylcellulose-based medium (HCC-3434; Stem Cell Technologies) supplemented with recombinant mouse (rm) stem cell factor (rmSCF, 50 ng/ml), mouse rm interleukin-3 (rmIL-3, 10 ng/ml), recombinant human (rh) interleukin-6 (rhIL-6, 10 ng/ml) and rh erythropoietin (rhEpo, 3 U/ml). Colonies were scored according to standard criteria after 12-14 days of incubation at 37°C in a humidified atmosphere of 5% CO₂ in air (Humphries, R.K.et al., Blood, 53:746-763, 1979.).

**Long-term culture-initiating cell (LTC-IC) assay.** LTC-IC were assessed in bulk cultures (Carlo-Stella C, et al. Blood. 1999;93:3973-82). Briefly, test cells (5 - 8 x 10⁶) were resuspended in complete medium (Myelocult™ 5100, Stem Cell Technologies) and seeded into cultures containing a feeder layer of irradiated (2,000 cGy) murine AFT024 cells (kindly provided by Dr. K. Moore, Princeton University, Princeton, NJ, USA) (Moore KA, et al., Blood. 1997;89:4337-47).

Complete medium consisted of alpha-medium supplemented with FBS (12.5%), horse serum (12.5%), L-glutamine (2 mM), 2-mercaptoethanol (10⁻⁴ M), inositol (0.2 mM), folic acid (20 µM) plus freshly dissolved hydrocortisone (10⁻⁶ M). Cultures were fed weekly by replacement of half of the growth medium with fresh complete medium. After 4 weeks in culture, nonadherent cells and adherent cells harvested by trypsinization were pooled, washed, and assayed together for clonogenic cells in methylcellulose cultures. The total number of clonogenic cells (i.e., CFU-GEMM plus BFU-E plus CFU-GM) present in 4-week-old LTC provides a relative measure of the number of LTC-IC originally present in the test suspension. Absolute LTC-IC values were calculated by dividing the total number of clonogenic cells by 4, which is the average output of clonogenic cells per LTC-IC (Sutherland HJ, et al.,Blood. 1989;74:1563-70).

### EXAMPLE 1

**Table 1 - WBC counts in mice treated with rmP1GF and/or rhG-CSF**

| Mobilization Regimen* | WBC/µL blood | |
|---|---|---|
| | Median (range) | Mean ± SD |
| PBS/MSA | 2,000 (850 - 4,000) | 2,165 ± 929 |
| rhG-CSF (10 µg/d) | 6,000 (5,200 - 21,650) | 9,577 ± 5,575 |
| rmP1GF (5 µg/d) | 2,450 (1,350 - 2,950) | 2,450 ± 141 |
| rhG-CSF (10 µg/d) + rmP1GF (2.5 µg/d) | 5,600 (4,600 - 13,700) | 7,040 ± 3,778 |
| rhG-CSF (10 µg/d) + rmP1GF (5 µg/d) | 9,500 (4,800 - 18,400) | 9,980 ± 5,715 |

| | | |
|---|---|---|
| * BALB/c mice were injected IP for 5 days with either PBS/MSA, rhG-CSF alone (10 µg/d), or a combination of rhG-CSF (10 µg/d) with rmP1GF (2.5 - 5 µg/d). Blood samples were collected 2 hours after the last injection of rmP1GF and/or rhG-CSF. | | |

### EXAMPLE 2

**Table 2 - Frequency of circulating CFCs in mice treated with rmP1GF and/or rhG-CSF**

| Mobilization Regimen* | CFCs/10⁵ MNCs | |
|---|---|---|
| | Median (range) | Mean ± SD |
| PBS/MSA | 7 (2 - 15) | 8 ± 3 |
| rhG-CSF (10 µg/d) | 76 (51 -148) | 82 ± 29 |
| rmP1GF (5 µg/d) | 8 (7 - 9) | 8 ± 1 |
| rhG-CSF (10 µg/d) + rmP1GF (2.5 µg/d) | 115 (93 - 184) | 130 ± 37 |
| rhG-CSF (10 µg/d) + rmP1GF (5 µg/d) | 195 (113 - 253) | 180 ± 58 |

| | | |
|---|---|---|
| * BALB/c mice were injected IP for 5 days with either PBS/MSA, rhG-CSF alone (10 µg/d), or a combination of rhG-CSF (10 µg/d) with rmPlGF (2.5 - 5 µg/d). Blood samples were collected 2 hours after the last injection of rmPlGF and/or rhG-CSF. CFCs include granulocyte-macrophage CFC (CFU-GM), erythroid burst-forming unit (BFU-E), and multipotent CFC (CFU-Mix). CFC data are derived from quadruplicate cultures on samples from each animal. | | |

### EXAMPLE 3

**Table 3 - Absolute number of circulating CFCs in mice treated with rmPlGF and/or rhG-CSF**

| Mobilization Regimen* | CFCs per ml Blood | |
|---|---|---|
| | Median (range) | Mean ± SD |
| PBS/MSA | 57 (9 - 288) | 81 ± 75 |
| rhG-CSF (10 µg/d) | 3,129 (1,042 - 5,518) | 2,977 ± 1,126 |
| rmPlGF (5 µg/d) | 96 (87 - 105) | 96 ± 13 |
| rhG-CSF (10 µg/d) + rmPlGF (2.5 µg/d) | 2,568 (1,480 - 5,885) | 3,198 ± 1,928 |
| rhG-CSF (10 µg/d) + rmPlGF (5 µg/d) | 6,143 (2,486 - 11,520) | 6,015 ± 3,674 |

| | | |
|---|---|---|
| * BALB/c mice were injected IP for 5 days with either PBS/MSA, rhG-CSF alone (10 µg/d), or a combination of rhG-CSF (10 µg/d) with rmPlGF (2.5 - 5 µg/d). Blood samples were collected 2 hours after the last injection of rmPlGF and/or rhG-CSF. CFCs include granulocyte-macrophage CFC (CFU-GM), erythroid burst-forming unit (BFU-E), and multipotent CFC (CFU-Mix). CFC data are derived from quadruplicate cultures on samples from each animal. The absolute number of circulating CFCs in blood is a function of the frequency of CFC multiplied by the total number of MNCs per ml blood. | | |

### EXAMPLE 4

**Table 4 - Absolute number of circulating LTC-ICs in mice treated with rmPlGF and/or rhG-CSF**

| Mobilization Regimen* | LTC-ICs per ml Blood | |
|---|---|---|
| | Median (range) | Mean ± SD |
| PBS/MSA | 7 (3 - 29) | 9 ± 5 |
| rhG-CSF (10 µg/d) | 194 (57 - 337) | 208 ± 98 |
| rmPlGF (5 µg/d) | 4(3-5) | 4 ± 2 |
| rhG-CSF (10 µg/d) + rmPlGF (2.5 µg/d) | 565 (279 - 852) | 565 ± 405 |
| rhG-CSF (10 µg/d) + rmPlGF (5 µg/d) | 1,173 (852 - 2,070) | 1,365 ± 364 |

| | | |
|---|---|---|
| * BALB/c mice were injected IP for 5 days with either PBS/MSA, rhG-CSF alone (10 µg/d), or a combination of rhG-CSF (10 µg/d) with rmPlGF (2.5 - 5 µg/d). Blood samples were collected 2 hours after the last injection of rmPlGF and/or rhG-CSF. The absolute number of circulating LTC-IC was assayed in bulk cultures. Test cells (5 - 8 x 10⁶) were seeded into cultures containing a feeder layer of irradiated murine AFT024 cells. After 4 weeks in culture, nonadherent cells and adherent cells harvested by trypsinization were pooled, washed, and assayed together for clonogenic cells. The total number of clonogenic cells (i.e., CFU-Mix plus BFU-E plus CFU-GM) present in 4-week-old LTC provides a relative measure of the number of LTC-IC originally present in the test suspension. The absolute number of circulating LTC-ICs in blood is a function of the frequency of LTC-ICs multiplied by the total number of MNCs per ml blood. | | |

### EXAMPLES 5-11 - mobilizing effects of PIGF/G-CSF combination in a non-human primate model

### MATERIALS AND METHODS

**Experimental design.** A cohort of Rhesus Monkeys (n = 4) was initially mobilized with G-CSF alone (100 µg/kg/day, SC, for 5 days) (**cycle 1**), and after a 6-week wash-out period, received a second mobilization therapy consisting of rhP1GF (130 µg/kg, IV, for 5 days) plus rhG-CSF (100 µg/kg/day, SC, for 5 days) (**cycle 2**). After an additional 6-week wash-out period, a third mobilization cycle consisting of rhPlGF (260 µg/kg, IV, for 5 days) plus rhG-CSF (100 µg/kg/day, SC, for 5 days) (**cycle 3**) was administered to the same cohort of monkeys. According to the study designs, the kinetics of mobilization following cycle 1 served as intra-monkey control to assess the mobilization following cycles 2 and 3.

**Mobilization parameters.** We analyzed the mobilization kinetics of white blood cells (WBCs), as well as frequency and absolute numbers of committed colony-forming cells (CFCs), high-proliferative potential progenitors (HPP-CFCs), and long-term culture-initiating cells (LTC-ICs). Mobilization parameters were analyzed daily during treatment (days 1 to 5), and 3 and 5 days post-cessation of therapy. Peripheral blood samples were obtained from the femoral vein of anesthetized primates (ketamin, 10 mg/kg, intramuscularly) using aseptic techniques.

**WBC counts.** WBC counts were performed using EDTA-anticoagulated blood and an automated counter (ADVIA 120, Bayer, Milano, Italy, EU).

**CFC and HPP-CFC assays.** Total CFCs [i.e., granulocyte-macrophage colony-forming units (CFU-GM), erythroid burst-forming units (BFU-E), and multilineage (granulocyte, erythrocyte, macrophage, megakaryocyte) CFU (CFU-GEMM)] and HPP-CFCs were assayed by using heparinized blood according to a previously described technique (41, 42). Briefly, mononuclear cells (MNCs) obtained by centrifugation on a Ficoll discontinuous gradient (density = 1.077 g/ml) were plated (1 x 10⁴ to 2 x 10⁵ per ml) in quadruplicate in 35-mm Petri dishes in methylcellulose-based medium (HCC-4100, Stem Cell Technologies, Vancouver, Canada) supplemented with recombinant human stem cell factor (rhSCF, 50 ng/ml, Stem Cell Technologies), interleukin-3 (rhIL-3, 20 ng/ml, Stem Cell Technologies), interleukin-6 (rhIL-6, 20 ng/ml, Stem Cell Technologies), rhG-CSF (20 ng/ml, Stem Cell Technologies), granulocyte-macrophage colony-stimulating factor (rhGM-CSF, 20 ng/ml, Stem Cell Technologies), and erythropoietin (rhEpo, 3 U/ml, R&D Systems Inc., Abingdon, United Kingdom). CFCs were scored after 12-14 days of incubation (37°C, 5% CO₂) according to standard criteria. HPP-CFCs, defined as macroscopically visible colonies of >1 mm in diameter of compact colony growth, were scored after 28 days of incubation from methylcellulose cultures supplemented with rhSCF (50 ng/ml), rhIL-3 (20 ng/ml), rhIL-6 (20 ng/ml), rhG-CSF (20 ng/ml), rhGM-CSF (20 ng/ml), and rhEpo (3 U/ml) (43). The absolute number of circulating CFCs or HPP-CFCs in blood is a function of the frequency of CFCs or HPP-CFCs multiplied by the total number of MNCs per ml blood.

**LTC-IC assays.** The frequency of LTC-ICs was assessed under limiting dilution conditions (44). Briefly, serial dilutions of test cells (2 x 10⁵ to 3 x 10³) were resuspended in 150 µL complete medium (Myelocult™ 5100, Stem Cell Technologies) consisting of alpha-medium supplemented with fetal bovine serum (12.5%), horse serum (12.5%), L-glutamine (2 mM), 2-mercaptoethanol (10⁻⁴ M), inositol (0.2 mM), folic acid (20 µM) plus freshly dissolved hydrocortisone (10⁻⁶ M) and plated in 96-well flat-bottom plates. For each test cell dose, 16 to 22 replicates were plated. Test cells were seeded into plates containing a feeder layer of irradiated (8,000 cGy) murine M2-10B4 cells (3 x 10⁴/cm2, kindly provided by Dr. C. Eaves, Terry Fox Laboratory, Vancouver, Canada) engineered by retroviral gene transfer to produce human IL-3 and G-CSF (45). Cultures were fed weekly, by replacement of half of the growth medium with fresh complete medium. After 5 weeks in culture, nonadherent and adherent cells from individual wells were harvested by trypsinization, washed and assayed together for the growth of CFCs. After 12 to 14 days of incubation, cultures were scored as positive

(≥1 colony) or negative (no colony) and the LTC-IC frequencies were calculated by using L-Calc software (Stem Cell Technologies). The absolute numbers of circulating LTC-IC were assessed in bulk cultures (46). Briefly, test cells (5 - 8 x 10⁶) were resuspended in complete medium and seeded into cultures containing a feeder layer of irradiated murine M2-10B4 cells (3 x 10⁴/cm²). After 5 weeks in culture, nonadherent cells and adherent cells harvested by trypsinization were pooled, washed, and assayed together for clonogenic cells. The total number of clonogenic cells (i.e., CFU-GEMM plus BFU-E plus CFU-GM) present in 5-week-old LTC provides a relative measure of the number of LTC-IC originally present in the test suspension. Absolute LTC-IC values were calculated by dividing the total number of clonogenic cells by 4, which is the average output of clonogenic cells per LTC-IC.

### EXAMPLE 5

**Circulating WBCs.** A 5-day administration of rhG-CSF alone induced an average 5-fold increment in the mean (±SD) numbers of WBCs, as compared to pretreatment values. Addition of 130 or 260 µg/kg rhPlGF to rhG-CSF resulted in a modest increase of WBC values detected on day 5 of treatment.

**Table 5 - WBC counts in Rhesus monkeys treated with rhG-CSF alone or rhPlGF plus rhG-CSF**

| | **WBC counts per µL blood *** | | |
|---|---|---|---|
| | **Cycle 1** | **Cycle 2** | **Cycle 3** |
| Day | rhG-CSF (100 µg/kg/day, SC, for 5 days) | rhPlGF (130 µg/kg, IV, for 5 days) + rhG-CSF (100 µg/kg/day, SC, for 5 days) | rhPlGF (260 µg/kg, IV, for 5 days) + rhG-CSF (100 µg/kg/day, SC, for 5 days) |
| 1 | 8,708 ± 2,458 | 13,498 ± 5,514 | 8,370 ± 1,585 |
| 2 | 31,313 ± 3,889 | 24,533 ± 2,789 | 41,180 ± 7,364 |
| 3 | 40,600 ± 6,274 | 35,388 ± 2,207 | 44,085 ± 6,588 |
| 4 | 43,055 ± 6,562 | 39,440 ± 6,744 | 37,960 ± 3,598 |
| 5 | 43,523 ± 13,790 | 60,040 ± 9,508 | 49,048 ± 7,120 |
| 8 | 14,363 ± 4,163 | 23,073 ± 9,017 | 17,783 ± 5,964 |
| 10 | 12,145 ± 5,421 | 16,398 ± 8,314 | 11,150 ± 2,915 |

| | | | |
|---|---|---|---|
| * Rhesus monkeys (n = 4) received three mobilization cycles separated by a 6-week washout period. Mobilization was elicited at cycle 1 by rhG-CSF alone (100 µg/kg/day, SC, day 1 - 5), at cycle 2 by a combination of rhPlGF (130 µg/kg, IV, day 1 - 5) plus rhG-CSF (100 µg/kg/day, SC, day 1 -5), and at cycle 3 by a combination of rhPlGF (260 µg/kg, IV, day 1 - 5) plus rhG-CSF (100 µg/kg/day, SC, day 1 -5). WBC counts were analyzed daily during treatment (days 1 to 5), as well as 3 and 5 days post-cessation of therapy. Data are expressed as mean ± SD. | | | |

### EXAMPLE 6

**Frequency of CFCs.** As compared to baseline values, the mean frequencies of blood CFCs (per 10⁵ MNCs) detected at peak were increased by 19-, 53-, and 52-fold under rhG-CSF alone, rhG-CSF/rhPlGF (130 µg/kg), and rhG-CSF/rhPlGF (260 µg/kg), respectively. As compared to rhG-CSF alone, the combined rhPlGF/rhG-CSF treatment induced a 2-fold increase of CFC frequency on the day of peak.

**Table 6 - Frequency of circulating CFCs in Rhesus monkeys treated with rhG-CSF alone or rhPlGF plus rhG-CSF**

| | **CFCs/10⁵ MNCs *** | | |
|---|---|---|---|
| | **Cycle 1** | **Cycle 2** | **Cycle 3** |
| Day | rhG-CSF (100 µg/kg/day, SC, for 5 days) | rhPlGF (130 µg/kg, IV, for 5 days) + rhG-CSF (100 µg/kg/day, SC, for 5 days) | rhPlGF (260 µg/kg, IV, for 5 days) + rhG-CSF (100 µg/kg/day, SC, for 5 days) |
| 1 | 6 ± 1 | 4 ± 1 | 5 ± 3 |
| 2 | 4 ± 2 | 9 ± 1 | 19 ± 8 |
| 3 | 9 ± 1 | 39 ± 13 | 48 ± 26 |
| 4 | 114 ± 51 | 213 ± 87 | 245 ± 151 |
| 5 | 63 ± 26 | 196 ± 26 | 261 ± 83 |
| 8 | 66 ± 11 | 40 ± 11 | 60 ± 39 |
| 10 | 10 ± 7 | 19 ± 10 | 21 ± 18 |

| | | | |
|---|---|---|---|
| * Rhesus monkeys (n = 4) received three mobilization cycles separated by a 6-week washout period. Mobilization was elicited at cycle 1 by rhG-CSF alone (100 µg/kg/day, SC, day 1 - 5), at cycle 2 by a combination of rhPlGF (130 µg/kg, IV, day 1 - 5) plus rhG-CSF (100 µg/kg/day, SC, day 1 -5), and at cycle 3 by a combination of rhPlGF (260 µg/kg, IV, day 1 - 5) plus rhG-CSF (100 µg/kg/day, SC, day 1 -5). CFCs were analyzed daily during treatment (days 1 to 5), as well as 3 and 5 days post-cessation of therapy. Data are expressed as mean ± SD. CFCs include granulocyte-macrophage CFC (CFU-GM), erythroid burst-forming unit (BFU-E), and multipotent CFC (CFU-Mix). CFC data are derived from quadruplicate cultures on samples from each animal. | | | |

### EXAMPLE 7

**Absolute values of CFCs.** Absolute numbers of circulating CFCs in blood were calculated as a function of the frequency of CFCs multiplied by the total number of MNCs per ml blood. As compared to baseline values, treatment with rhG-CSF alone, rhG-CSF/rhPlGF (130 µg/kg), and rhG-CSF/rhPlGF (260 µg/kg) resulted in a 85- 335- and 358-fold increase of CFCs, respectively. At cycles 2 and 3, the peak levels of CFCs were increased by 4- and 5-fold over cycle 1 (rhG-CSF alone).

**Table 7 - Absolute numbers of circulating CFCs in Rhesus Monkeys treated with rhG-CSF alone or rhPlGF plus rhG-CSF**

| | | **CFCs per ml blood *** | |
|---|---|---|---|
| | **Cycle 1** | **Cycle 2** | **Cycle 3** |
| Day | rhG-CSF(100 µg/kg/day, SC, for 5 days) | rhPlGF (130 µg/kg, IV, for 5 days) + rhG-CSF (100 µg/kg/day, SC, for 5 days) | rhPlGF (260 µg/kg, IV, for 5 days) + rhG-CSF (100 µg/kg/day, SC, for 5 days) |
| | 1 134 ± 9 | 138 ± 38 | 170 ± 129 |
| 2 | 344 ± 207 | 724 ± 254 | 6,552 ± 4,365 |
| 3 | 472 ± 60 | 6,420 ± 4,775 | 9,634 ± 7,006 |
| 4 | 11,406 ± 4,093 | 32,347 ± 14,206 | 53,002 ± 25,250 |
| 5 | 5,397 ± 3,074 | 46,283 ± 8,287 | 60,777 ± 8,563 |
| 8 | 3,952 ± 2,666 | 4,532 ± 3,714 | 3,719 ± 1,899 |
| 10 | 224 ± 164 | 448 ± 168 | 943 ± 994 |

| | | | |
|---|---|---|---|
| * Rhesus monkeys (n = 4) received three mobilization cycles separated by a 6-week washout period. Mobilization was elicited at cycle 1 by rhG-CSF alone (100 µg/kg/day, SC, day 1 - 5), at cycle 2 by a combination of rhPlGF (130 µg/kg, IV, day 1 - 5) plus rhG-CSF (100 µg/kg/day, SC, day 1 -5), and at cycle 3 by a combination of rhPlGF (260 µg/kg, IV, day 1 - 5) plus rhG-CSF (100 µg/kg/day, SC, day 1 -5). CFCs were analyzed daily during treatment (days 1 to 5), as well as 3 and 5 days post-cessation of therapy. Data are expressed as mean ± SD. CFCs include granulocyte-macrophage CFC (CFU-GM), erythroid burst-forming unit (BFU-E), and multipotent CFC (CFU-Mix). CFC data are derived from quadruplicate cultures on samples from each animal. The absolute number of circulating CFCs in blood is a function of the frequency of CFC multiplied by the total number of MNCs per ml blood. | | | |

### EXAMPLE 8

**Frequency of HPP-CFCs.** As compared to baseline values, the mean frequencies of blood HPP-CFCs (per 10⁵ MNCs) detected on day 5 of mobilization were increased by 5-, and 12-fold under rhG-CSF alone or rhG-CSF/rhPlGF (130 µg/kg), respectively. As compared to rhG-CSF alone, the combined rhPlGF/rhG-CSF treatment induced a 2-fold increase of HPP-CFC frequency on the day of peak.

**Table 8 - Frequency of circulating HPP-CFCs in Rhesus monkeys treated with rhG-CSF alone or rhPlGF plus rhG-CSF**

| | **HPP-CFCs/10⁵ MNCs *** | |
|---|---|---|
| | **Cycle 1** | **Cycle 2** |
| Day | rhG-CSF (100 µg/kg/day, SC, for 5 days) | rhPlGF (130 µg/kg, IV, for 5 days) +rhG-CSF (100 µg/kg/day, SC, for 5 days) |
| 1 | 4 ± 1 | 3 ± 1 |
| 2 | 6 ± 1 | 3 ± 1 |
| 3 | 13 ± 4 | 11 ± 3 |
| 4 | 15 ± 4 | 27 ± 10 |
| 5 | 20 ± 9 | 37 ± 8 |
| 8 | 18 ± 6 | 6 ± 4 |
| 10 | 6 ± 1 | 5 ± 4 |

| | | |
|---|---|---|
| * Rhesus monkeys (n = 4) received three mobilization cycles separated by a 6-week washout period. Mobilization was elicited at cycle 1 by rhG-CSF alone (100 µg/kg/day, SC, day 1 - 5), at cycle 2 by a combination of rhPlGF (130 µg/kg, IV, day 1 - 5) plus rhG-CSF (100 µg/kg/day, SC, day 1 -5), and at cycle 3 by a combination of rhPlGF (260 µg/kg, IV, day 1 - 5) plus rhG-CSF (100 µg/kg/day, SC, day 1 -5). HPP-CFCs were analyzed daily during treatment (days 1 to 5), as well as 3 and 5 days post-cessation of therapy. Data are expressed as mean ± SD. HPP-CFC data are derived from quadruplicate cultures on samples from each animal. | | |

### EXAMPLE 9

**Absolute values of HPP-CFCs.** The absolute number of HPP-CFCs per ml blood detected on day 5 of rhG-CSF therapy was 17-fold higher than pretreatment values. Monkeys receiving the combined rhG-CSF/rhPlGF (130 µg/kg) treatment showed a 158-fold increase of HPP-CFCs as compared to baseline values. At cycle 2, the level of day-5 HPP-CFCs was increased by 5-fold over cycle 1.

**Table 9 - Absolute numbers of circulating HPP-CFC in Rhesus Monkeys treated with rhG-CSF alone or rhPlGF plus rhG-CSF**

| | **HPP-CFCs per ml blood *** | |
|---|---|---|
| | **Cycle 1** | **Cycle 2** |
| Day | rhG-CSF (100 µg/kg/day, SC, for 5 days) | rhPlGF (130 µg/kg, IV, for 5 days) + rhG-CSF (100 µg/kg/day, SC, for 5 days) |
| 1 | 96 ± 17 | 54 ± 49 |
| 2 | 493 ± 218 | 258 ± 34 |
| 3 | 683 ± 155 | 1,709 ± 989 |
| 4 | 1,521 ± 332 | 3,883 ± 1,309 |
| 5 | 1,593 ± 405 | 8,557 ± 1,142 |
| 8 | 998 ± 541 | 603 ± 384 |
| 10 | 121 ± 52 | 121 ± 87 |

| | | |
|---|---|---|
| * Rhesus monkeys (n = 4) received three mobilization cycles separated by a 6-week washout period. Mobilization was elicited at cycle 1 by rhG-CSF alone (100 µg/kg/day, SC, day 1 - 5), at cycle 2 by a combination of rhPlGF (130 µg/kg, IV, day 1 - 5) plus rhG-CSF (100 µg/kg/day, SC, day 1 -5), and at cycle 3 by a combination of rhPlGF (260 µg/kg, IV, day 1 - 5) plus rhG-CSF (100 µg/kg/day, SC, day 1 -5). HPP-CFC counts were analyzed daily during treatment (days 1 to 5), as well as 3 and 5 days post-cessation of therapy. Data are expressed as mean ± SD. HPP-CFCs data are derived from quadruplicate cultures on samples from each animal. The absolute number of circulating HPP-CFCs in blood is a function of the frequency of HPP-CFCs multiplied by the total number of MNGs-per ml blood. | | |

### EXAMPLE 10

**Frequency of LTC-ICs.** Analysis of the LTC-IC frequency by a limiting dilution assay showed that the combined administration of rhPlGF (130 µg/kg) and rhG-CSF resulted in an average increase the LTC-IC frequency by 11-fold (1 in 5,829 vs 1 in 64,064 cells), as compared to rhG-CSF alone.

**Table 10 - Frequency of circulating LTC-ICs in Rhesus Monkeys receiving a 5-day course of rhG-CSF alone or rhPlGF plus rhG-CSF**

| Animal No. | Mobilization Regimen | LTC-IC Frequency (mean)* | 95% CI | | LTC-IC 10⁵ per MNCs |
|---|---|---|---|---|---|
| | | | Lower Frequency | Upper Frequency | |
| | | | | | |
| 1 | rhG-CSF | 1/84,265 | 1/69,209 | 1/102,598 | 1.2 |
| 2 | rhG-CSF | 1/65,835 | 1/54,341 | 1/79,761 | 1.5 |
| 3 | rhG-CSF | ne ** | ne | ne | ne |
| 4 | rhG-CSF | 1/42,091 | 1/34,837 | 1/50,854 | 2.4 |
| | | | | | |
| 1 | rhPlGF (130 µg/kg) + rhG-CSF | 1/4,009 | 1/5,977 | 1/2,689 | 24.9 |
| 2 | rhPlGF (130 µg/kg) + rhG-CSF | 1/7,562 | 1/11,100 | 1/5,152 | 13.2 |
| 3 | rhPlGF (130 µg/kg) + rhG-CSF | ne | ne | ne | ne |
| 4 | rhPlGF (130 µg/kg) + rhG-CSF | 1/5,916 | 1/8,725 | 1/4,011 | 16.9 |

| | | | | | |
|---|---|---|---|---|---|
| * The frequency of LTC-IC was assayed under limiting dilution conditions using the murine M2-10B4 cell line as stromal layer. Blood samples were collected on day 5 of mobilization therapy. Serial dilutions of test cells (2 x 10⁵ to 3 x 10³) were cultured for 5 weeks and 16 to 22 replicates were plated for each test cell dose. After 5 weeks, nonadherent and adherent cells from individual wells were assayed for clonogenic cells and the LTC-IC frequencies were calculated using Poisson statistics and the method of maximum likelihood. | | | | | |

### EXAMPLE 11

**Absolute values of LTC-ICs.** Under rhG-CSF alone, absolute numbers of circulating LTC-ICs were increased by 53-fold on day 4 of treatment as compared to baseline values. The combined rhG-CSF/rhPlGF (130 µg/kg) treatment increased LTC-ICs by 389-fold as compared to pretreatment values, and by 15-fold as compared to rhG-CSF alone.

**Table 11 - Absolute numbers of circulating LTC-ICs in Rhesus Monkeys treated with rhG-CSF alone or rhPlGF plus rhG-CSF**

| | **LTC-ICs per ml blood *** | |
|---|---|---|
| | **Cycle 1** | **Cycle 2** |
| Day | rhG-CSF (100 µg/kg/day, SC, for 5 days) | rhPlGF (130 µg/kg, IV, for 5 days) + rhG-CSF (100 µg/kg/day, SC, for 5 days) |
| 1 | 4 ± 7 | 8 ± 5 |
| 2 | 92 ± 43 | 56 ± 20 |
| 3 | 111 ± 30 | 624 ± 340 |
| 4 | 211 ± 41 | 742 ± 176 |
| 5 | 130 ± 25 | 3,115 ± 988 |
| 8 | 63 ± 22 | 533 ± 270 |
| 10 | 6 ± 2 | 112 ± 40 |

| | | |
|---|---|---|
| * Rhesus monkeys (n = 4) received three mobilization cycles separated by a 6-week washout period. Mobilization was elicited at cycle 1 by rhG-CSF alone (100 µg/kg/day, SC, day 1 - 5), at cycle 2 by a combination of rhPlGF (130 µg/kg, IV, day 1 - 5) plus rhG-CSF (100 µg/kg/day, SC, day 1 -5), and at cycle 3 by a combination of rhPlGF (260 µg/kg, IV, day 1 - 5) plus rhG-CSF (100 µg/kg/day, SC, day 1 -5). LTC-IC counts were analyzed daily during treatment (days 1 to 5), as well as 3 and 5 days post-cessation of therapy. Data are expressed as mean ± SD derived from quadruplicate cultures on samples from each animal at each time point. The absolute number of circulating LTC-IC was assayed in bulk cultures. Test cells (5 - 8 x 10⁶) were seeded into cultures containing a feeder layer of irradiated murine M2-10B4 cells. After 5 weeks in culture, nonadherent cells and adherent cells harvested by trypsinization were pooled, washed, and assayed together for clonogenic cells. The total number of clonogenic cells (i.e., CFU-Mix plus BFU-E plus CFU-GM) present in 5-week-old LTC provides a relative measure of the number of LTC-IC originally present in the test suspension. The absolute number of circulating LTC-ICs in blood is a function of the frequency of LTC-ICs multiplied by the total number of MNCs per ml blood. | | |

### BIBLIOGRAPHY

1. Gianni AM. High-dose chemotherapy and autotransplants: a time for guidelines. Ann Oncol. 1997;8:933-5.
2. Demirer T, Bensinger WI, Buckner CD. Peripheral blood stem cell mobilization for high-dose chemotherapy. J Hematother. 1999;8:103-13.
3. Gianni AM, Bregni M, Siena S, et al. High-dose chemotherapy and autologous bone marrow transplantation compared with MACOP-B in aggressive B-cell lymphoma. N Engl J Med. 1997;336:1290-7.
4. Ferme C, Mounier N, Divine M, et al. Intensive salvage therapy with high-dose chemotherapy for patients with advanced Hodgkin's disease in relapse or failure after initial chemotherapy: results of the Groupe d'Etudes des Lymphomes de l'Adulte H89 Trial. J Clin Oncol. 2002;20:467-75.
5. Barlogie B. High-dose therapy and innovative approaches to treatment of multiple myeloma. Semin Hematol. 2001;38(2 Suppl 3):21-7.
6. Korbling M, Przepiorka D, Huh YO, et al. Allogeneic blood stem cell transplantation for refractory leukemia and lymphoma: potential advantage of blood over marrow allografts. Blood. 1995;85:1659-1665.
7. Schmitz N, Bacigalupo A, Hasenclever D, et al. Allogeneic bone marrow transplantation vs filgrastim-mobilised peripheral blood progenitor cell transplantation in patients with early leukaemia: first results of a randomised multicentre trial of the European Group for Blood and Marrow Transplantation. Bone Marrow Transplant. 1998;21:995-1003.
8. Appelbaum FR. Choosing the source of stem cells for allogeneic transplantation: no longer a peripheral issue. Blood. 1999;94:381-383.
9. Bensinger WI, Martin PJ, Storer B, et al. Transplantation of bone marrow as compared with peripheral-blood cells from HLA-identical relatives in patients with hematologic cancers. N Engl J Med. 2001;344:175-181.
10. Anderlini P, Korbling M, Dale D, et al. Allogeneic blood stem cell transplantation: considerations for donors. Blood. 1997;90:903-908.
11. Aversa F, Tabilio A, Terenzi A, et al. Successful engraftment of T-cell-depleted haploidentical "three-loci" incompatible transplants in leukemia patients by addition of recombinant human granulocyte colony-stimulating factor-mobilized peripheral blood progenitor cells to bone marrow inoculum. Blood. 1994;84:3948-3955.
12. Haas R, Mohle R, Fruhauf S, et al. Patient characteristics associated with successful mobilizing and autografting of peripheral blood progenitor cells in malignant lymphoma. Blood. 1994;83:3787-94.
13. Bensinger WI, Longin K, Appelbaum F, et al. Peripheral blood stem cells (PBSCs) collected after recombinant granulocyte colony stimulating factor (rhG-CSF): an analysis of factors correlating with the tempo of engraftment after transplantation. Br J Haematol. 1994;87:825-31.
14. Watts MJ, Sullivan AM, Jamieson E, et al. Progenitor-cell mobilization after low-dose cyclophosphamide and granulocyte colony-stimulating factor: an analysis of progenitor-cell quantity and quality and factors predicting for these parameters in 101 pretreated patients with malignant lymphoma. J Clin Oncol. 1997;15:535-46.
15. Matsunaga T, Sakamaki S, Kohgo Y, Ohi S, Hirayama Y, Niitsu Y. Recombinant human granulocyte colony-stimulating factor can mobilize sufficient amounts of peripheral blood stem cells in healthy volunteers for allogeneic transplantation. Bone Marrow Transplant. 1993;11:103-108.
16. Kroger N, Renges H, Sonnenberg S, et al. Stem cell mobilisation with 16 µg/kg vs 10 µg/kg of G-CSF for allogeneic transplantation in healthy donors. Bone Marrow Transplant. 2002;29:727-730.
17. Basara N, Schmetzer B, Blau IW, et al. Lenograstim-mobilized peripheral blood progenitor cells in volunteer donors: an open label randomized split dose escalating study. Bone Marrow Transplant. 2000;25:371-376.
18. Engelhardt M, Bertz H, Afting M, Waller CF, Finke J. High-versus standard-dose filgrastim (rhG-CSF) for mobilization of peripheral-blood progenitor cells from allogeneic donors and CD34+ immunoselection. J Clin Oncol. 1999;17:2160-2172.
19. Siena S, Schiavo R, Pedrazzoli P, Carlo-Stella C. Therapeutic relevance of CD34+ cell dose in blood cell transplantation for cancer therapy. J Clin Oncol. 2000;18:1360-77.
20. Dreger P, Kloss M, Petersen B, et al. Autologous progenitor cell transplantation: prior exposure to stem cell-toxic drugs determines yield and engraftment of peripheral blood progenitor cell but not of bone marrow grafts. Blood. 1995;86:3970-8.
21. Weaver CH, Hazelton B, Birch R, et al. An analysis of engraftment kinetics as a function of the CD34 content of peripheral blood progenitor cell collections in 692 patients after the administration of myeloablative chemotherapy. Blood. 1995;86:3961-9.
22. Tarella C, Di Nicola M, Caracciolo D. High-dose ara-C with autologous peripheral blood progenitor cell support induces a marked progenitor cell mobilization: an indication for patients at risk for low mobilization. Bone Marrow Transplant. 2002;30:725-32.
23. Anderlini P, Przepiorka D, Seong D, et al. Clinical toxicity and laboratory effects of granulocyte-colony-stimulating factor (filgrastim) mobilization and blood stem cell apheresis from normal donors, and analysis of charges for the procedures. Transfusion. 1996;36:590-595.
24. Anderlini P, Przepiorka D, Huh Y, et al. Duration of filgrastim mobilization and apheresis yield of CD34+ progenitor cells and lymphoid subsets in normal donors for allogeneic transplantation. Br J Haematol. 1996;93 :940-942.
25. Grigg AP, Roberts AW, Raunow H, et al. Optimizing dose and scheduling of filgrastim (granulocyte colony-stimulating factor) for mobilization and collection of peripheral blood progenitor cells in normal volunteers. Blood. 1995;86:4437-4445.
26. Aversa F, Tabilio A, Velardi A, et al. Treatment of high-risk acute leukemia with T-cell-depleted stem cells from related donors with one fully mismatched HLA haplotype. N Engl J Med. 1998;339:1186-1193.
27. Miflin G, Charley C, Stainer C, Anderson S, Hunter A, Russell N. Stem cell mobilization in normal donors for allogeneic transplantation: analysis of safety and factors affecting efficacy. Br J Haematol. 1996;95:345-348.
28. Anderlini P, Przepiorka D, Seong C, et al. Factors affecting mobilization of CD34+ cells in normal donors treated with filgrastim. Transfusion. 1997;37:507-512.
29. de la Rubia J, Arbona C, de Arriba F, et al. Analysis of factors associated with low peripheral blood progenitor cell collection in normal donors. Transfusion. 2002;42:4-9.
30. Craddock CF, Nakamoto B, Andrews RG, Priestley GV, Papayannopoulou T. Antibodies to VLA4 integrin mobilize long-term repopulating cells and augment cytokine-induced mobilization in primates and mice. Blood. 1997;90:4779-88.
31. King AG, Horowitz D, Dillon SB, et al. Rapid mobilization of murine hematopoietic stem cells with enhanced engraftment properties and evaluation of hematopoietic progenitor cell mobilization in rhesus monkeys by a single injection of SB-251353, a specific truncated form of the human CXC chemokine GROβ. Blood. 2001;97:1534-42.
32. Pruijt JF, van Kooyk Y, Figdor CG, Lindley IJ, Willemze R, Fibbe WE. Anti-LFA-1 blocking antibodies prevent mobilization of hematopoietic progenitorcells induced by interleukin-8. Blood. 1998;91:4099-105.
33. Carlo-Stella C, Di Nicola M, Magni M, et al. Defibrotide in combination with granulocyte colony-stimulating factor significantly enhances the mobilization of primitive and committed peripheral blood progenitor cells in mice. Cancer Res. 2002;62:6152-7.
34. Koc ON, Gerson SL, Cooper BW, et al. Randomized cross-over trial of progenitor-cell mobilization: high-dose cyclophosphamide plus granulocyte colony-stimulating factor (G-CSF) versus granulocyte-macrophage colony-stimulating factor plus G-CSF. J Clin Oncol. 2000;18:1824-30.
35. Rosenfeld CS, Bolwell B, LeFever A, et al. Comparison of four cytokine regimens for mobilization of peripheral blood stem cells: IL-3 alone and combined with GM-CSF or G-CSF. Bone Marrow Transplant. 1996; 17):179-83.
36. Bishop MR, Jackson JD, O'Kane-Murphy B, et al. Phase I trial of recombinant fusion protein PIXY321 for mobilization of peripheral-blood cells. J Clin Oncol. 1996;14:2521-6.
37. Shpall EJ, Wheeler CA, Turner SA, et al. A randomized phase 3 study of peripheral blood progenitor cell mobilization with stem cell factor and filgrastim in high-risk breast cancer patients. Blood. 1999;93:2491-501.
38. Facon T, Harousseau JL, Maloisel F, et al. Stem cell factor in combination with filgrastim after chemotherapy improves peripheral blood progenitor cell yield and reduces apheresis requirements in multiple myeloma patients: a randomized, controlled trial. Blood. 1999;94:1218-25.
39. Brasel K, McKenna HJ, Charrier K, Morrissey PJ, Williams DE, Lyman SD. Flt3 ligand synergizes with granulocyte-macrophage colony-stimulating factor or granulocyte colony-stimulating factor to mobilize hematopoietic progenitor cells into the peripheral blood of mice. Blood. 1997;90:3781-8.
40. Hattori K, Heissig B, Wu Y, et al. Placental growth factor reconstitute hematopoiesis by recruiting VEGFR1+ stem cells from bone marrow microenvironment. Nature Med. 2002;8:841-9.
41. MacVittie TJ, Farese AM, Davis TA, Lind LB, McKeam JP. Myelopoietin, a chimeric agonist of human interleukin 3 and granulocyte colony-stimulating factor receptors, mobilizes CD34+ cells that rapidly engraft lethally x-irradiated nonhuman primates. Exp Hematol. 1999; 27:1557-68.
42. Carlo-Stella C, Di Nicola M, Longoni P, Milani R, Milanesi M, Guidetti A, Haanstra K, Jonker M, Cleris L, Magni M, Formelli F, Gianni AM. Mobilization of primitive and committed hematopoietic progenitors in nonhuman primates treated with defibrotide and recombinant human granulocyte colony-stimulating factor. Exp Hematol. 2004; 32:68-75.
43. Craddock CF, Nakamoto B, Andrews RG, Priestley GV, Papayannopoulou T. Antibodies to VLA-4 integrin mobilize long-term repopulating cells and augment cytokine-induced mobilization in primates and mice. Blood. 1997; 90:4779-88.
44. Lemieux ME, Rebel VI, Lansdorp PM, Eaves CJ. Characterization and purification of a primitive hematopoietic cell type in adult mouse marrow capable of lymphomyeloid differentiation in long-term marrow "switch" cultures. Blood. 1995;86:1339-1347.
45. Sutherland HJ, Eaves CJ, Lansdorp PM, Tacker JD, Hogge DE. Differential regulation of primitive human hematopoietic cells in long-term cultures maintained on genetically engineered murine stromal cells. Blood. 1991;78:666-72.
46. Carlo-Stella C, Regazzi E, Sammarelli G, et al. Effects of the tyrosine kinase inhibitor AG957 and an Anti-Fas receptor antibody on CD34+ chronic myelogenous leukemia progenitor cells. Blood. 1999;93:3973-82.
47. Sutherland HJ, Eaves CJ, Eaves AC, Dragowska AC, Lansdorp PM. Characterization and partial purification of human marrow cells capable of initiating long-term hematopoiesis in vitro. Blood. 1989; 74:1563-70.

## Claims

1. Combined pharmaceutical preparation containing G-CSF and P1GF as the active substances, for use in stimulating the mobilization of peripheral blood progenitor cells (PBPCs) for increasing the feasibility and efficacy of organ or cell transplantation and of chemo-radiotherapy protocols in tumor patients.

2. Combined pharmaceutical preparation for a use according to claim 1, wherein said cell transplantation is an autologous or allogeneic stem cell transplantation carried out in patients with non-Hodgkin lymphoma (NHL), relapsed Hodgkin lymphoma (*EL*), multiple myeloma, or the recovery phase following myelosuppressive chemotherapy transplantation.

3. Combined preparation for a use according to claim 1, wherein G-CSF and P1GF are simultaneously or separately administered to said patient or subject.

4. Combined preparation for a use according to claims 1-3, for parenteral administration.

5. Combined preparation for a use according to claims 1-4, containing recombinant hG-CSF and rhPlGF.

6. Combined preparation for a use according to claims 1-5, containing from 1 to 150µg/kg G-CSF and from 10 to 300 µg/kg P1GF.

## Patentansprüche

1. Kombiniertes pharmazeutisches Präparat, das G-CSF und P1GF als aktive Substanzen enthält, zur Verwendung bei der Stimulation der Mobilisierung peripherer Blutprogenitorzellen (PBPC's) zur Verbesserung der Durchführbarkeit und der Wirksamkeit von Organ- oder Zelltransplantationen und von Chemo-Radiotherapieprotokollen bei Tumorpatienten.

2. Kombiniertes pharmazeutisches Präparat für eine Verwendung gemäß Anspruch 1, wobei besagte Zelltransplantation eine autologe oder allogene Stammzellentransplantation ist, die ausgeführt wird bei Patienten mit Non-Hodgkin-Lymphom(NHL), rezidivem Hodgkin-Lymphom (EL), multiplem Myelom oder in der Genesungsphase, die auf myelosuppressive Chemotherapie-Transplantation folgt.

3. Kombiniertes Präparat für eine Verwendung gemäß Anspruch 1, wobei G-CSF und P1GF gleichzeitig oder getrennt voneinander dem Patienten oder Probanden verabreicht werden.

4. Kombiniertes Präparat für eine Verwendung gemäß einem der Ansprüche 1 - 3 zur parenteralen Verabreichung.

5. Kombiniertes Präparat für eine Verwendung gemäß einem der Ansprüche 1 - 4, das hG-CSF undrhP1GF enthält.

6. Kombiniertes Präparat für eine Verwendung gemäß einem der Ansprüche 1 - 5, das von 1 bis 150 µg/kg G-CSF und von 10 bis 300 µg/kg P1GF enthält.

## Revendications

1. Préparation pharmaceutique combinée contenant G-CSF et P1GF en tant que les substances actives, destinée à une utilisation pour stimuler la mobilisation de cellules souches de sang périphérique (PBPC) pour augmenter la faisabilité et l'efficacité d'une transplantation d'organes ou de cellules et des protocoles de chimio-radiothérapie chez des patients atteints de tumeurs.

2. Préparation pharmaceutique combinée destinée à une utilisation selon la revendication 1, où ladite transplantation de cellules est une transplantation de cellules souches autologue ou allogénique réalisée chez des patients atteints d'un lymphome non Hodgkinien (NHL), d'une rechute d'un lymphome Hodgkinien (EL), d'un myélome multiple, ou en phase de convalescence suivant une transplantation à chimiothérapie myélosuppressive.

3. Préparation combinée destinée une utilisation selon la revendication 1, dans laquelle G-CSF et P1GF sont administrés simultanément ou séparément audit patient ou sujet.

4. Préparation combinée destinée à une utilisation selon les revendications 1 à 3, pour une administration par voie parentérale.

5. Préparation combinée destinée à une utilisation selon les revendications 1 à 4, contenant hG-CSF et rhPlGF recombinants.

6. Préparation combinée destinée à une utilisation selon les revendications 1 à 5, contenant de 1 à 150 µg/kg de G-CSF et de 10 à 300 µg/kg de PlGF.
